# EUROPEAN PATENT APPLICATION

(11) **EP 4 781 896 A1**
(43) Date of publication of application: **29.07.2026**
(21) Application number: 25153494.7
(22) Date of filing: 23.01.2025
(51) Int. Cl.: A61B 3/10, A61F 9/008

(54) **METHOD FOR PROVIDING CONTROL DATA FOR AN OPHTHALMIC DEVICE FOR CORRECTION OF HIGH ORDER ABERRATIONS OF AN EYE, CONTROL DEVICE, TREATMENT APPARATUS, COMPUTER PROGRAM AND NON-TRANSITORY COMPUTER-READABLE MEDIUM**

(71) Applicant: Schwind Eye-Tech-Solutions GmbH, 63801 Kleinostheim (DE)
(72) Inventor: ARBA MOSQUERA, Samuel, 63739 Aschaffenburg (DE); Gatinel, Damien, 75006 Paris (FR); Malet, Jacques, 75014 Paris (FR)
(74) Representative: Hofstetter, Schurack & Partner

(57) **Abstract**

The invention relates to a method for providing control data for an ophthalmic device for correction of high order aberrations of an eye. The method comprises the steps of determining a wavefront; determining high degree wavefront aberration coefficients by using a decomposition of the wavefront into a Gatinel-Malet basis, wherein the Gatinel-Malet basis is formed by a low degree Gatinel-Malet basis that comprises low degree aberration polynomials that are orthogonal to each other and a high degree Gatinel-Malet basis that comprises the high degree aberration polynomials that are orthogonal to each other, wherein the aberration polynomials of the low degree Gatinel-Malet basis are non-orthogonal to the aberration polynomials of the high degree Gatinel-Malet basis; determining correction data for the eye by using the determined high degree wavefront aberration coefficients; and providing control data for the ophthalmic device, comprising the determined correction data.

## Description

The present invention relates to a method for providing control data for an ophthalmic device for correction of high order aberrations of an eye. In addition, the invention relates to a treatment apparatus with at least one eye surgical laser and at least one control device for performing the method. Furthermore, the present invention relates to a computer program, including instructions that cause the treatment apparatus to execute the method, as well as a non-transitory computer-readable medium, on which the computer program is stored.

Treatment apparatuses and methods for controlling ophthalmological lasers for correcting an optical visual disorder and/or pathologically or unnaturally altered areas of the cornea are known in the prior art. Therein, pulsed lasers and a beam focusing device may, for example, be formed such that laser pulses effect a photodisruption and/or photoablation in a focus located within the organic tissue to remove tissue, in particular a tissue lenticule, from the cornea.

For planning the treatment with an ophthalmic device wavefront measurements of the eye may be utilized to determine different components of visual disorder. For example, a wavefront may be decomposed into Zernike polynomials of multiple orders, wherein each of the Zernike polynomials may describe a refraction or aberration. In other words, Zernike polynomials may be used to represent wavefronts, which in turn describe imaging errors of optical systems. In ophthalmic optics, Zernike polynomials are typically used for planning eye surgical treatments.

These Zernike polynomials are mutually orthogonal, and the polynomials are divided into order terms, wherein Zernike polynomials equal or lower than two are denoted as low order or low degree Zernike polynomials and these with an order higher or equal than three are denoted as high order polynomials. For example, low order Zernike polynomials may comprise a tilt and defocus and higher order polynomials may comprise aberrations like trefoil, coma and spherical aberrations.

However, the use of Zernike polynomials has some drawbacks with regard of low degree Zernike terms that are embedded into the higher order polynomials. Therefore, an interference of low degree Zernike polynomials may occur when planning for a correction of high order aberrations. To avoid this coupling between low and high order aberrations, a new polynomial basis is provided, which is described in the publication "Polynomial Decomposition Method for Ocular Wavefront Analysis" (Damien Gatinel, Jacques Malet, and Laurent Dumas; Journal of the Optical Society of America, 2018, Vol. 35, No. 12, 2035-2045), wherein this new basis for polynomial decomposition will be referred to as Gatinel-Malet basis in the following. In particular, the Gatinel-Malet basis provides low degree and high degree aberration polynomials, wherein the polynomials in the low degree Gatinel-Malet basis are orthogonal to each other, and the polynomial of a high degree Gatinel-Malet basis are orthogonal to each other. However, the polynomials of the low degree and high degree Gatinel-Malet basis are non-orthogonal to each other, which opens up further possibilities for providing high degree polynomials without the affection from low order terms.

The object of the present invention is to find a robust strategy to provide improved correction data to correct for high order aberrations of an eye.

This object is solved by the independent claims. Advantageous embodiments of the invention are specified in the respective dependent claims, the following description and the figures.

A first aspect of the present invention relates to a method for providing control data for an ophthalmic device for correction of high order aberrations of an eye, wherein the following steps are performed by a control device. As steps the method comprises determining a wavefront from a predetermined wavefront measurement, determining high degree wavefront aberration coefficients in dependence on the determined wavefront by using a decomposition of the wavefront into a Gatinel-Malet basis, wherein the Gatinel-Malet basis is formed by a low degree Gatinel-Malet basis that comprises low degree aberration polynomials and a high degree Gatinel-Malet basis that comprises the high degree aberration polynomials, wherein the aberration polynomials of the low degree Gatinel-Malet basis are orthogonal to each other and the aberration polynomials of the high degree Gatinel-Malet basis are orthogonal to each other, and wherein the aberration polynomials of a low degree Gatinel-Malet basis are non-orthogonal to the aberration polynomials of the high degree Gatinel-Malet basis. Moreover, the method comprises the steps of determining correction data for the eye by using the determined high degree wavefront aberration coefficients and providing control data for the ophthalmic device, comprising the determined correction data.

In other words, high degree wavefront aberration coefficients for correction of high order aberrations may be determined from a wavefront by using the Gatinel-Malet basis. The Gatinel-Malet basis may be formed by low degree Gatinel-Malet basis that includes low degree aberration polynomials and a high degree Gatinel-Malet basis that includes high degree aberration polynomials. The aberration polynomials of low degree and high degree Gatinel-Malet basis may be orthogonal (orthonormal) on their own, but non-orthogonal to each other. Thus, the aberration polynomials of the high degree Gatinel-Malet basis do not contain lower degree terms.

The wavefront that is to be analyzed may be provided by using a wavefront sensor, for example, a Hartmann-Shack sensor. The control device that performs the method steps may be formed as a computer, in particular, comprising a processor or a microprocessor that is configured to automatically or semiautomatically perform the determining of correction data by using the wavefront from the predetermined wavefront measurement as input, wherein the wavefront is processed by using the Gatinel-Malet basis, in particular the low degree and high degree Gatinel-Malet basis. Moreover, further examination data may be utilized by the control device to determine the high degree wavefront aberration coefficients. Finally, the control data for the ophthalmic device may be generated, comprising the determined correction data.

The ophthalmic device may be treatment apparatus comprising an ophthalmic laser, such as a laser configured for photodisruption and/or photoablation to correct a visual disorder, in particular a high order aberration of the eye by removing tissue using the determined correction data. The control data generated by the control device may comprise control signals for controlling the eye surgical laser, a scanner and/or a focusing device to deflect a laser beam into the cornea.

The advantage of the invention is that better correction data may be obtained. In particular, high order aberrations of an eye may be corrected without risking to pronounced interference with low degree terms that makes customized treatment planning more intuitive and precise.

The invention also includes embodiments by which additional advantages arise.

Fitting the given wavefront directly to the Gatinel-Malet basis is not yet sufficient to achieve a complete improvement of correcting high order aberrations since this corresponds to a univocal mathematical transformation respectively change of basis to the Gatinel-Malet basis and thus numbers would be different but the derived refraction would unlikely match the actual clinical refraction. Therefore, different approaches to remove the clinical refraction from the wavefront prior to fitting the modified wavefront to the high degree Gatinel-Malet basis need to be provided that are presented in the following embodiments.

In one embodiment, the method comprises the steps of determining manifest refraction values from predetermined subjective examination data, determining low degree manifest aberration coefficients by fitting the manifest refraction values to the low degree aberration polynomials of the low degree Gatinel-Malet basis, wherein the low degree manifest aberration coefficients of the low degree aberration polynomials represent a low degree manifest aberration wavefront, subtracting the obtained low degree manifest aberration wavefront from the determined wavefront to obtain a high degree aberration wavefront, decomposing the high degree aberration wavefront by using the high degree aberration polynomials of the high degree Gatinel-Malet basis to obtain high degree wavefront aberration coefficients, and determining the correction data for the eye by using the obtained high degree wavefront aberration coefficients. In other words, in this embodiment, manifest refraction values from predetermined subjective examination data may be used as additional input to determine the correction data, in particular to remove the clinical refraction from the wavefront prior to fitting the modified wavefront to the high degree Gatinel-Malet basis. Subjective examination data, in particular the manifest refraction values, may be determined during an examination, wherein a combination of lenses are provided to a patient and the patient indicates which combination provides the best vision. The parameters of the lenses used in this subjective measurement represent the manifest refraction values. For example, values in diopters may be provided, for example values for sphere, cylinder and axis. Because these manifest refraction values represents low order aberrations of the eye, the manifest refraction values may be converted into low degree manifest aberration coefficients by fitting the manifest refraction values to the low degree aberration polynomials of the low degree Gatinel-Malet basis. That is, the obtained manifest refraction values may be converted to a wavefront representation, wherein the wavefront representation of the manifest refraction values is denoted as "low degree manifest aberration wavefront" in the following. This low degree manifest aberration wavefront may then be subtracted from the full wavefront to obtain a "high degree aberration wavefront". The high degree aberration wavefront may then be decomposed by using the high degree aberration polynomials of the high degree Gatinel-Malet basis to obtain the high degree wavefront aberration coefficients, by which the correction data to correct for high order aberrations of the eye may be determined. Thereby, the manifest or clinical refraction derived from the low degree aberration polynomials may be removed from the whole wavefront and fitting the resulting wavefront to the high degree aberration polynomials of the Gatinel-Malet basis provides the high degree wavefront aberration coefficients that are free from an influence of the low degree terms. This embodiments provides the advantage that high order aberrations may be corrected more precise and in a simple way.

In another embodiment, the method comprises the steps of determining manifest refraction values from predetermined subjective examination data, determining low degree manifest aberration coefficients by fitting the manifest refraction values to the low degree aberration polynomials of the low degree Gatinel-Malet basis, determining low degree wavefront aberration coefficients from the wavefront by using the low degree aberration polynomials of the low degree Gatinel-Malet basis, calculating a difference between the low degree manifest aberration coefficients and the low degree wavefront aberration coefficients to obtain low degree delta coefficients, converting the low degree delta coefficients into the high degree Gatinel-Malet basis to obtain high degree delta coefficients, adding the obtained high degree delta coefficients to the high degree wavefront aberration coefficients to obtain manifest corrected high degree wavefront aberration coefficients, and determining the correction data by using the obtained manifest corrected high degree wavefront aberration coefficients. In other words, first two different low degree aberration coefficients are determined, one from the manifest refraction values and the other from the wavefront. Then, the difference between the low degree manifest aberration coefficients (manifest refraction derived) and the low degree wavefront aberration coefficients (wavefront derived) may be calculated, wherein the difference is denoted as "low degree delta coefficients" in the following. A difference between the low degree coefficients may arise from low order terms of the manifest refraction that are present in high order aberrations. Therefore, the remaining low degree delta coefficients may be converted into the high degree Gatinel-Malet basis to obtain "high degree delta coefficients". The conversion of the low degree delta coefficients into the high degree Gatinel-Malet basis may be provided by fitting this difference to the high degree Gatinel-Malet basis so that the high degree components that may be present in the subjective examination data are received. The obtained high degree delta coefficients may then be added to the high degree wavefront aberration coefficients (wavefront derived) to obtain manifest corrected high degree wavefront aberration coefficients from which the correction data may be derived. This embodiment may provide the same results as the previous embodiment, however, numerically a slightly different result may arrive due to a low-pass filter associated with the Gatinel-Malet basis fit.

In another embodiment, the method comprises the steps of determining manifest refraction values from predetermined subjective examination data, determining low degree manifest aberration coefficients by fitting the manifest refraction values to the low degree aberration polynomials of the low degree Gatinel-Malet basis, determining low degree wavefront aberration coefficients from the wavefront by using the low degree aberration polynomials of the low degree Gatinel-Malet basis, calculating a difference between the low degree manifest aberration coefficients and the low degree wavefront aberration coefficients to obtain low degree delta coefficients, fitting the low degree delta coefficients by using the aberration polynomials of the low and high degree Gatinel-Malet basis with the constraint that the low degree fit coefficients of the low degree aberration polynomials result in Zero or a predefined range around Zero, to obtain high degree delta coefficients, adding the obtained high degree delta coefficients to the high degree wavefront aberration coefficients to obtain manifest corrected high degree wavefront aberration coefficients, and determining the correction data by using the obtained manifest corrected high degree wavefront coefficients. In other words, again two low degree aberration coefficients from different sources may be determined. In particular, the low degree manifest aberration coefficients may be determined from the manifest refraction values by fitting them to the low degree aberration polynomials, and the low degree wavefront aberration coefficients may be determine by fitting the wavefront from the predetermined wavefront measurement to the low degree aberration polynomials of a low degree Gatinel-Malet basis. In a similar way to the previous embodiments, the difference between the low degree manifest aberration coefficients and the low degree wavefront aberration coefficients is calculated to obtain the low degree delta coefficients. The low degree delta coefficients are then fitted to the low degree aberration polynomials of the low degree Gatinel-Malet basis as well as the high degree aberration polynomials of the high degree Gatinel-Malet basis with the fit-constraint that the low degree fit coefficients of the low degree aberration polynomials result in Zero or result in a predefined range around Zero. For example, the constraint may be preset as boundary condition for the fit. Consequently, only the high order components of the manifest refraction values are maintained by the fit, which are referred to here as high degree delta coefficients. These may be added to the high degree wavefront aberration coefficients that are obtained from the wavefront of the predetermined wavefront measurement. Thus, the addition results in the manifest corrected high degree wavefront aberration coefficients. The manifest corrected high degree wavefront aberration coefficients may then be used to determine the correction data for the correction of high order aberrations of the eye.

In another embodiment, the method comprises the steps of fitting the determined wavefront by using the low and high degree aberration polynomials of the Gatinel-Malet basis to obtain low and high degree aberration coefficients, wherein the high degree aberration coefficients of the high degree aberration polynomials represent a high degree aberration wavefront, fitting the high degree aberration wavefront by using the low degree polynomials of the low degree Gatinel-Malet basis to obtain coefficients of a low degree portion of the high degree aberration wavefront, subtracting the coefficients of the low degree portion from the high degree wavefront aberration coefficients to obtain corrected high degree wavefront aberration coefficients, and determining the correction data using the obtained corrected high degree wavefront aberration coefficients. In other words, the wavefront that is determined from the predetermined wavefront measurement is fitted to the low degree aberration polynomials of a low degree Gatinel-Malet basis as well as to the high degree aberration polynomials of the high degree Gatinel-Malet basis resulting in low and high degree aberration coefficients. Hereby, the high degree aberration coefficients of the high degree aberration polynomials are representing a high degree aberration wavefront. The high degree aberration wavefront that may be influenced by a portion of low order aberrations may then be fitted to the low degree polynomials of the low degree Gatinel-Malet basis to extract coefficients of the low degree portion from the high degree aberration wavefront. In particular, the coefficients of a low degree portion may be subtracted from the high degree wavefront aberration coefficients to obtain the corrected high degree wavefront aberration coefficients that are free of the low degree portion. The obtained corrected high degree wavefront aberration coefficients may then be used to determine the correction data to correct for high order aberrations of the eye.

In another embodiment, the method comprises the steps of determining manifest refraction values from predetermined subjective examination data, determining low degree manifest aberration coefficients by fitting the manifest refraction values to the low degree aberration polynomials of the low degree Gatinel-Malet basis, decomposing the wavefront by using only the low degree aberration polynomials of the low degree Gatinel-Malet basis to obtain low degree wavefront aberration coefficients, calculating a difference between the low degree manifest aberration coefficients and the low degree wavefront aberration coefficients to obtain low degree delta coefficients, fitting the wavefront to low and high degree aberration polynomials using the low and high degree Gatinel-Malet basis, wherein the low degree delta coefficients are accounted for in the fit as a dynamic fit constraint, and determining high order aberration correction data using the obtained high degree wavefront aberration coefficients. For example, the whole wavefront fit to the low degree polynomials of the low degree Gatinel-Malet basis (=low degree wavefront aberration coefficients) results in 2 diopters and the manifest refraction fit to the low degree polynomials of the low degree Gatinel-Malet basis (=low degree manifest aberration coefficients) results in 3 diopters, thus this difference is 1 diopter. The whole wavefront fit to the low and high degree polynomials may result in low degree wavefront aberration coefficients of 4 diopters, but the low degree coefficients shall include 5 diopters (4+1). Thus, to account for the low degree delta coefficients, these are added as constraint to the low and high degree wavefront coefficients. This means that the low degree delta coefficients sets limits within which the fit may vary.

In another embodiment, the method comprises the steps of determining manifest refraction values from predetermined subjective examination data, determining low degree manifest aberration coefficients by fitting the manifest refraction values to the low degree aberration polynomials of the low degree Gatinel-Malet basis, fitting the wavefront to low and high degree aberration polynomials using the low and high degree Gatinel-Malet basis with the constraint that the low degree fit coefficients of the low degree aberration polynomials results in the determined low degree manifest aberration coefficients, to obtain manifest corrected high degree wavefront coefficients, and determining the correction data by using the obtained manifest corrected high degree wavefront coefficients. In other words, in this embodiment, in the same way as in the previous embodiments, low degree manifest aberration coefficients from the manifest refraction values may be determined by fitting the manifest refraction values to the low degree aberration polynomials of the low degree Gatinel-Malet basis Furthermore, the wavefront that is provided from the predetermined wavefront measurement may be fitted to the low degree aberration polynomials of the low degree Gatinel-Malet basis as well as to the high degree aberration polynomials of the high degree Gatinel-Malet basis, wherein the fit is constrained such that the low degree fit coefficients of the low degree aberration polynomials result in the low degree manifest aberration coefficients that were obtained from the manifest refraction values. Thus, high degree coefficients are obtained that are decoupled from an influence of low order components determined from the manifest refraction (manifest corrected high degree wavefront aberration coefficients), wherein these obtained manifest corrected high degree wavefront aberration coefficients are used to derive the correction data to correct an eye for high order aberrations.

The method may include at least one additional step, which is executed if and only if an application case or an application situation occurs, which has not been explicitly described here. For example, the step may include the output of an error message and/or the output of a request for inputting a user feedback. Additionally or alternatively, it may be provided that a default setting and/or a predetermined initial state are adjusted.

A further aspect of the invention relates to a control device, which is configured to perform the steps of at least one embodiment the previously described method. Thereto, the control device may comprise a computing unit for electronic data processing such as for example a processor. The computing unit may include at least one microcontroller and/or at least one microprocessor. The computing unit may be configured as an integrated circuit and/or microchip. Furthermore, the control device may include an (electronic) data memory or a storage unit. A program code may be stored on the data memory, by which the steps of the respective embodiment of the respective method are encoded. The program code may include the control data for the respective laser. The program code may be executed by the computing unit, whereby the control device is caused to execute the respective embodiment. The control device may be formed as a control chip or control unit. The control device may for example be encompassed by a computer or computer cluster.

A further aspect of the invention relates to a treatment apparatus with at least one eye surgical or ophthalmological laser and a control device, which is configured to perform the steps of at least one embodiment of the previously described method. The respective laser may be configured to at least partially separate a predefined corneal volume, in particular with predefined interfaces, of a human or animal eye by optical breakdown, in particular at least partially separate it by photodisruption and/or to ablate corneal layers by (photo)ablation and/or to effect a laser-induced refractive index change in the cornea and/or the eye lens.

In a further embodiment of the treatment apparatus according to the invention, the laser may be suitable to emit laser pulses in a wavelength range between 300 nm and 1400 nm, for example between 900 nm and 1200 nm, at a respective pulse duration between 1 fs and 1 ns, for example between 10 fs and 10 ps, and a repetition frequency of greater than 10 kilohertz (kHz), for example between 100 kHz and 100 megahertz (MHz). The use of such lasers in the method according to the invention additionally has the advantage that the irradiation of the cornea does not have to be effected in a wavelength range below 300 nm. This range is subsumed by the term "deep ultraviolet" in the laser technology. Thereby, it is advantageously avoided that an unintended damage to the cornea is effected by these very short-wavelength and high-energy beams. Photodisruptive and/or ablative lasers of the type used here usually input pulsed laser radiation with a pulse duration between 1 fs and 1 ns into the corneal tissue. Thereby, the power density of the respective laser pulse required for the optical breakdown may be spatially narrowly limited such that a high incision accuracy is allowed in the generation of the interfaces. In particular, the range between 700 nm and 780 nm may also be selected as the wavelength range.

In a further embodiment of the treatment apparatus according to the invention, the control device may comprise at least one storage device for at least temporary storage of at least one control dataset, wherein the control dataset or datasets include(s) control data for positioning and/or for focusing individual laser pulses in the cornea; and may comprise at least one beam device for beam guidance and/or beam shaping and/or beam deflection and/or beam focusing of a laser beam of the laser.

A further aspect of the invention relates to a computer program. The computer program includes commands, which for example form a program code. The program code may include at least one control dataset with the respective control data for the respective laser. Upon execution of the program code by a computer or a computer cluster, it is caused to execute the previously described method or at least one embodiment thereof.

A further aspect of the invention relates to a non-transitory computer-readable medium (storage medium), on which the above mentioned computer program and the commands thereof, respectively, are stored. For executing the computer program, a computer or a computer cluster may access the computer-readable medium and read out the content thereof. The storage medium is for example formed as a data memory, in particular at least partially as a volatile or a non-volatile data memory. A non-volatile data memory may be a flash memory and/or an SSD (solid state drive) and/or a hard disk. A volatile data memory may be a RAM (random access memory). For example, the commands may be present as a source code of a programming language and/or as assembler and/or as a binary code.

Further features and advantages of one of the described aspects of the invention may result from the embodiments of another one of the aspects of the invention. Thus, the features of the embodiments of the invention may be present in any combination with each other if they have not been explicitly described as mutually exclusive.

In the following, additional features and advantages of the invention are described in the form of advantageous execution examples based on the figure(s). The features or feature combinations of the execution examples described in the following may be present in any combination with each other and/or the features of the embodiments. This means, the features of the execution examples may supplement and/or replace the features of the embodiments and vice versa. Thus, configurations are also to be regarded as encompassed and disclosed by the invention, which are not explicitly shown or explained in the figures, but arise from and may be generated by separated feature combinations from the execution examples and/or embodiments. Thus, configurations are also to be regarded as disclosed, which do not comprise all of the features of an originally formulated claim or extend beyond or deviate from the feature combinations set forth in the relations of the claims. To the execution examples, there shows:
- Fig. 1: a schematic representation of a treatment apparatus according to an exemplary embodiment;
- Fig. 2: a schematic method diagram for providing control data according to an exemplary embodiment;
- Fig. 3: a schematic method diagram for providing control data according to another exemplary embodiment;
- Fig. 4: a schematic method diagram for providing control data according to another exemplary embodiment;
- Fig. 5: a schematic method diagram for providing control data according to another exemplary embodiment;
- Fig. 6: a schematic method diagram for providing control data according to another exemplary embodiment;
- Fig. 7: a schematic method diagram for providing control data according to another exemplary embodiment.

In the figures, identical or functionally identical elements are provided with the same reference characters.

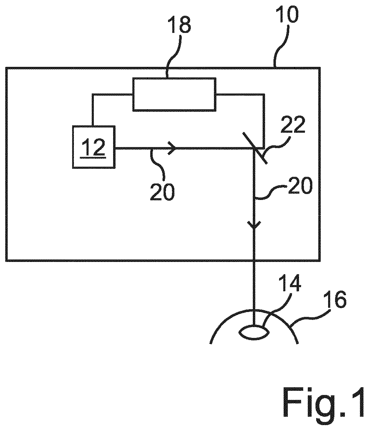

Fig. 1 shows a schematic representation of an ophthalmic device, in particular a treatment apparatus 10, with an eye surgical or ophthalmological laser 12 for removing a tissue or volume body 14 from a human or animal cornea 16 by photodisruption and/or ablation. The volume body 14 may for example represent a lenticule, which may be separated from the cornea 16 by the ophthalmological laser 12 for correcting a visual disorder. Correction data respectively a geometry of the volume body 14 to be removed may be provided by a control device 18, in particular in the form of control data, such that the laser 12 emits pulsed laser pulses in a pattern predefined by the control data into the cornea 16 of the eye to remove the volume body 14. Alternatively, the control device 18 may be a control device 18 external with respect to the treatment apparatus 10.

Furthermore, Fig. 1 shows that the laser beam 20 generated by the laser 12 may be deflected towards the cornea 16 by a beam deflection device 22 such as for example a rotation scanner, to remove the volume body 14. The beam deflection device 22 may also be controlled by the control device 18 to remove the volume body 14.

In particular, the illustrated laser 12 may be a photodisruptive and/or photoablative laser, which is formed to emit laser pulses in a wavelength range between 300 nanometers and 1400 nanometers, for example between 700 nanometers and 1200 nanometers, at a respective pulse duration between 1 femtosecond and 1 nanosecond, for example between 10 femtoseconds and 10 picoseconds, and a repetition frequency of greater than 10 kilohertz, for example between 100 kilohertz and 100 megahertz. In addition, the control device 18 optionally comprises a storage device (not illustrated) for at least temporary storage of at least one control dataset, wherein the control dataset or datasets include(s) control data for positioning and/or for focusing individual laser pulses in the cornea.

Visual defects that may be corrected by removing the volume body 14 from the cornea 16 may comprise high order aberrations. For example, spherical aberrations, coma and trefoil are such refractive errors of high order aberration that may be treated by the treatment apparatus 10. In order to obtain the correction data for these high order aberrations, it is common practice to determine a wavefront from a wavefront measurement and decomposed it into Zernike polynomials. In particular, by these Zernike polynomials the wavefront may be decomposed in low and high degree polynomials that represent the respective refractional error. However, because the Zernike polynomials are orthogonal to each other, the high degree Zernike polynomials comprise low order components that influence the correction of high order aberrations.

To take the effect of these low order components into account, the use of a new set of polynomials is suggested that may be utilized by the control device 18. In particular, aberration polynomials of a Gatinel-Malet basis have proven to be advantageous in this respect. The Gatinel-Malet basis may be divided into a low degree Gatinel-Malet basis that comprises low degree aberration polynomials and into a high degree Gatinel-Malet basis that comprises high degree aberration polynomials, wherein the aberration polynomials of the low degree Gatinel-Malet basis are orthogonal to each other and the aberration polynomials of the high degree Gatinel-Malet basis are orthogonal to each other. However, in contrast to Zernike polynomials, the aberration polynomials of the low degree Gatinel-Malet basis are non-orthogonal to the aberration polynomials of the high degree Gatinel-Malet basis. Thereby, a better distinction between the low and high order aberrations may be achieved. However, it is not efficient to directly decompose or fit the wavefront to the Gatinel-Malet basis to remove the high order portions from the high degree polynomials, and different approaches are necessary. These approaches are explained in the following with help of the figures, wherein the steps of these process charts may be performed by the control device 18 of the treatment apparatus 10.

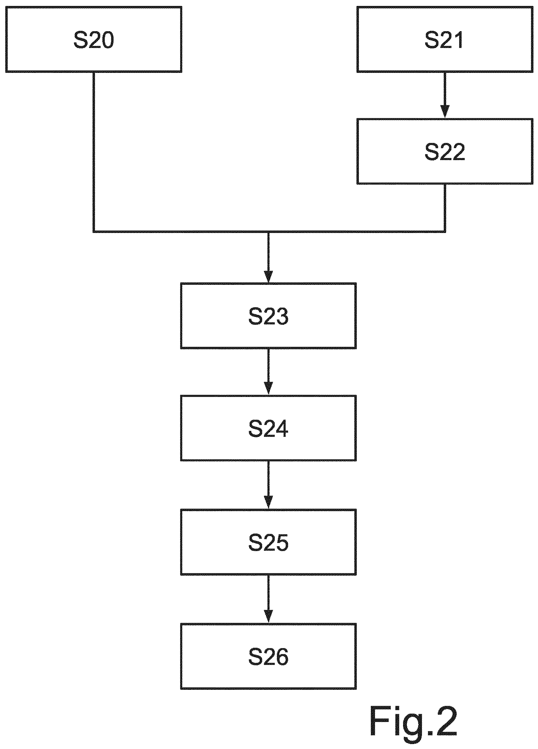

In Fig. 2, a method for providing control data for an ophthalmic device, in particular a treatment apparatus 10, for correction of high order aberrations according to one embodiment is shown. In a step S20, a wavefront may be determined from a predetermined wavefront measurement. For example, the wavefront may be measured by an aberrometer and the measured data may be provided to the control device 18 to receive the wavefront.

In a step S21, manifest refraction values of the eye may be determined form another measurement, in particular from predetermined subjective examination data. These manifest refraction values represent low order aberrations of the eye that may be measured by a phoropter. In a step S22, the determined manifest refraction values may be fitted to the low degree aberration polynomials of the low degree Gatinel-Malet basis to receive low degree manifest aberration coefficients. In particular, these low degree manifest aberration coefficients of the low degree aberration polynomials may be regarded as a wavefront (low degree manifest aberration wavefront).

In a step S23, this low degree manifest aberration wavefront may be subtracted from the whole wavefront obtained in step S20 to obtain a high degree aberration wavefront. Thus, the obtained high degree aberration wavefront is free of low order components.

Next, in a step S24 the high degree aberration wavefront may be decomposed respectively fitted by using the high degree aberration polynomials of the high degree Gatinel-Malet basis to obtain high degree wavefront aberration coefficients.

In a step S25, the obtained high degree wavefront aberration coefficients may be used to determine correction data to correct for the high order aberrations. This determination can be carried out in a conventional and known way

Finally, in a step S26 control data for the ophthalmic device, in particular the treatment apparatus 10, may be generated that are based on the correction data. The control data may comprise instructions by which the laser 12 may be controlled to create the volume body 14.

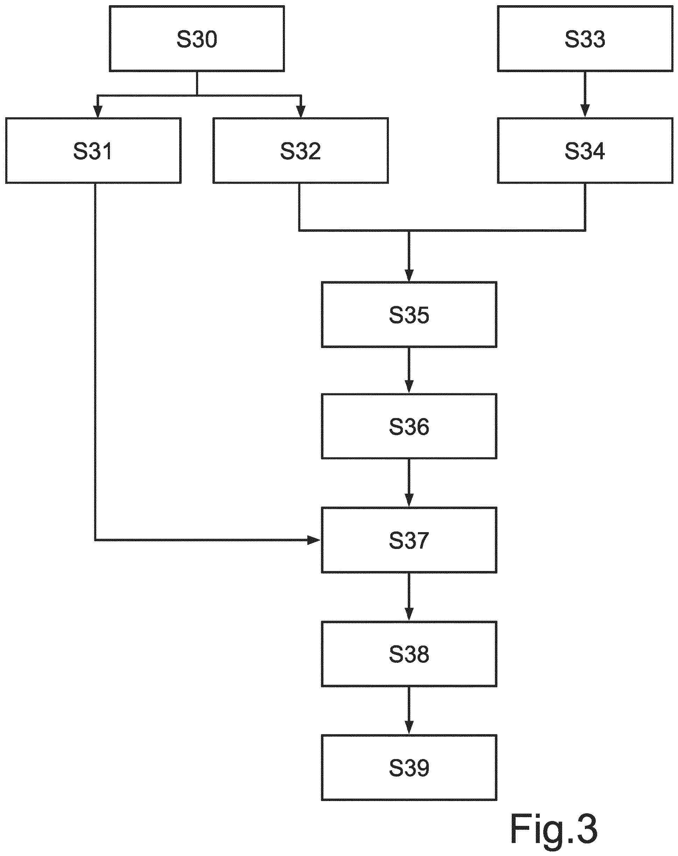

In Fig. 3, a process chart for the method according to another embodiment is shown that may be performed by the control device 18. In a step S30, a wavefront from a predetermined wavefront measurement may be obtained.

In a step S31, high degree wavefront aberration coefficients may be determined from the wavefront by using the high degree Gatinel-Malet basis. Additionally, in a step S32 low degree wavefront aberration coefficients are determined from the wavefront by using the low degree aberration polynomials of a low degree Gatinel-Malet basis.

From another measurement, in particular from predetermined subjective examination data, manifest refraction values may be determined in step S33 that are fitted to low degree aberration polynomials of the low degree Gatinel-Malet basis in a step S34 to receive low degree manifest aberration coefficients.

In a step S35, a difference is calculated between the low degree manifest aberration coefficients and the low degree wavefront aberration coefficients. This difference is hereinafter referred to as "low degree delta coefficients".

In a step S36, the obtained low degree delta coefficients are converted into the high degree Gatinel-Malet basis to obtain "high degree delta coefficients". Hereby, high degree portions that are present in the low degree coefficients are represented in the high degree delta coefficients.

In a step S37, the high degree wavefront aberration coefficients obtained in step S31 are added to the high degree delta coefficients obtained in step S36 to receive "manifest corrected high degree wavefront aberration coefficients".

In a step S38, the correction data may be determined by using the obtained manifest corrected high degree wavefront aberration coefficients, and in a step S39 the control data for the ophthalmic device 10 may be generated.

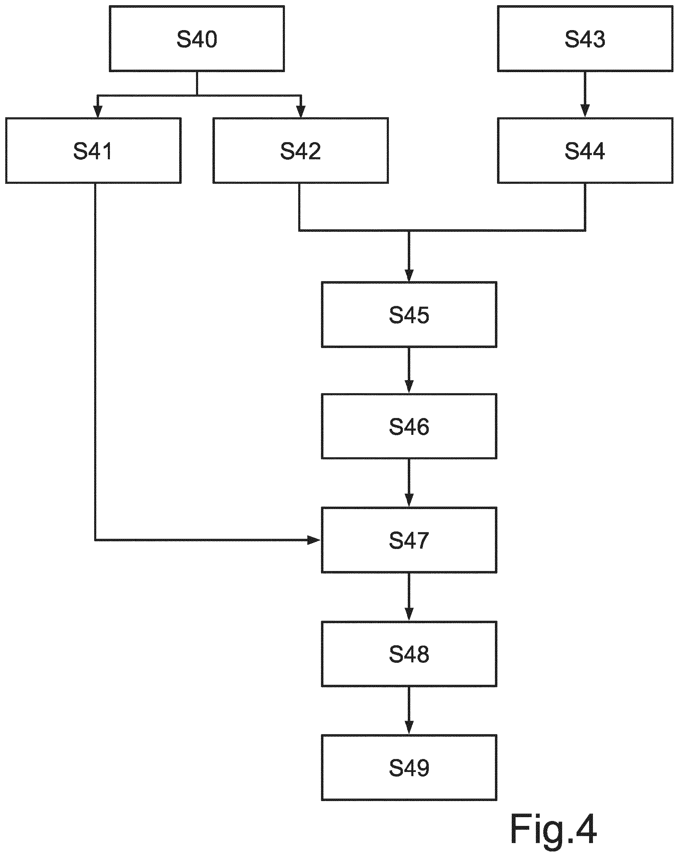

In Fig. 4, a process chart for the method according to another embodiment is shown that may be performed by the control device 18. In a step S40, the wavefront is determined from a predetermined wavefront measurement, wherein in a step S41 high degree wavefront aberration coefficients are determined from the wavefront by using the high degree aberration polynomials of the high degree Gatinel-Malet basis. Furthermore, in a step S42 low degree wavefront aberration coefficients are determined from the wavefront by using the low degree aberration polynomials of the low degree Gatinel-Malet basis.

In a step S43, manifest refraction values are determined from predetermined subjective examination data, and in a step S44 low degree manifest aberration coefficients are determined by fitting the manifest refraction values to the low degree aberration polynomials of the low degree Gatinel-Malet basis.

In a step S45, a difference between the low degree manifest aberration coefficients and the low degree wavefront aberration coefficients is calculated to obtain low degree delta coefficients.

Then, in a step S46, the low degree delta coefficients are fitted by using the aberration polynomials of low and high degree Gatinel-Malet basis with the constraint that low degree fit coefficients of the low degree aberration polynomials result in Zero or a predefined range around Zero to obtain high degree delta coefficients.

Then, in a step S47 the obtained high degree delta coefficients are added to the high degree wavefront aberration coefficients from step S41 to obtain manifest corrected high degree wavefront aberration coefficients.

Finally, in step S48 the correction data may be determined based on the obtained manifest corrected high degree wavefront coefficients, and in a step S49 the control data for the ophthalmic device 10 may be generated.

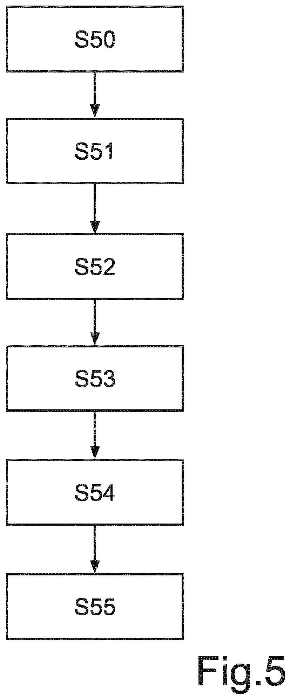

In Fig. 5, a process chart for the method according to another embodiment is shown that may be performed by the control device 18.

In step S50, the wavefront from the predetermined wavefront measurement may be determined. In a step S51, the determined wavefront may be fitted by using the low and high degree aberration polynomials of the Gatinel-Malet basis to obtain low and high degree aberration coefficients, wherein the high degree aberration coefficients of high degree aberration polynomials represent a high degree aberration wavefront.

In a step S52, the high degree aberration wavefront may be fitted by using the low degree polynomials of a low degree Gatinel-Malet basis to obtain coefficients of a low degree portion of the high degree aberration wavefront.

In step S53, the coefficients of the low degree portion are subtracted from the high degree wavefront aberration coefficients to obtain corrected high degree wavefront aberration coefficients.

Finally, in step S54 the correction data may be determined by using the obtained corrected high degree wavefront aberration coefficients, and in a step S55 the control data to control the ophthalmic device 10 may be generated in dependence of the correction data.

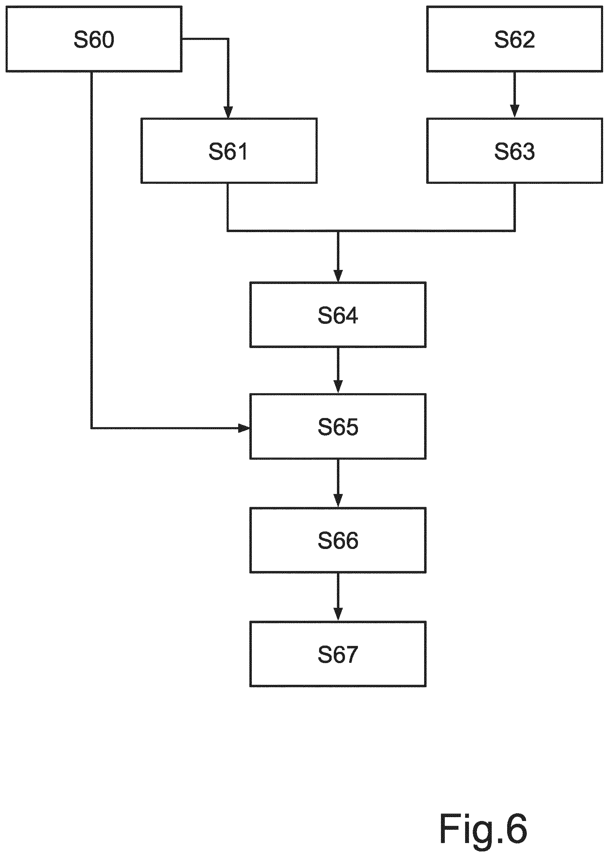

In Fig. 6, a process chart for the method according to another embodiment is shown that may be performed by the control device 18.

In a step S60, the wavefront from the predetermined wavefront measurement may be determined. In a step S61, the wavefront may be decomposed respectively fitted by using only the low degree aberration polynomials of the low degree Gatinel-Malet basis to obtain low degree wavefront aberration coefficients.

In a step S62, manifest refraction values may be determined from predetermined subjective examination data, and in a step S63 low degree manifest aberration coefficients may be determined by fitting the manifest refraction values to the low degree aberration polynomials of the low degree Gatinel-Malet basis.

In a step S64, a difference between the low degree manifest aberration coefficients and the low degree wavefront aberration coefficients may be calculated to obtain low degree delta coefficients.

In a step S65, the full wavefront may be fitted to low and high degree aberration polynomials using the low and high degree Gatinel-Malet basis, wherein the low degree delta coefficients from step S64 are accounted for in the fit as a dynamic fit constraint.

In step S66, the correction data to correct the high order aberrations may be obtained from the high degree aberration coefficients, and in a step S67 the control data to control the ophthalmic device 10 may be generated based on the correction data.

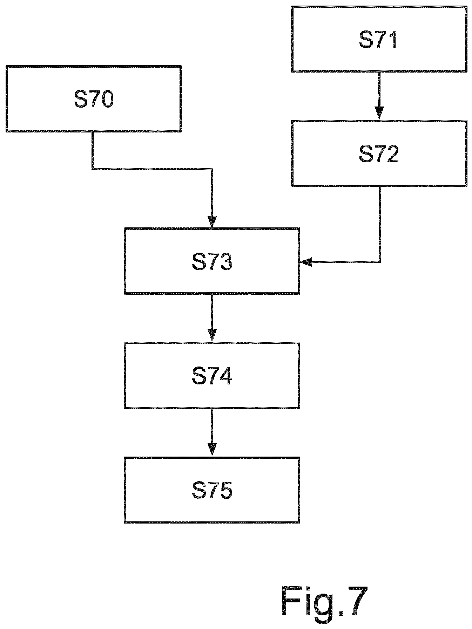

In Fig. 7, a process chart for the method according to another embodiment is shown that may be performed by the control device 18.

In a step S70, a wavefront may be determined form a predetermined wavefront measurement. For example, the wavefront may be measured by an aberrometer and the measured data may be provided to the control device 18 to receive the wavefront.

In a step S71, manifest refraction values of the eye may be determined form predetermined subjective examination data and in a step S72 low degree manifest aberration these manifest refraction values may be fitted to the low degree aberration polynomials of the low degree Gatinel-Malet basis to obtain low degree manifest aberration coefficients.

In a step S73, the wavefront from step S70 may be fitted to low and high degree aberration polynomials of the low and high degree Gatinel-Malet basis. Therefore, the low degree manifest aberration coefficients from step S72 may be utilized in the fit as constraint, so that the low degree fit coefficients of the wavefront fit result in these low degree manifest aberration coefficients. As a consequence, the high degree wavefront coefficients are free from manifest refraction terms or in other words the fit provides manifest corrected high degree wavefront coefficients.

In a step S74, the obtained manifest corrected high degree wavefront coefficients may be used to determine correction data to correct for the high order aberrations.

Finally, in a step S75 control data for the ophthalmic device, in particular the treatment apparatus 10, may be generated that are based on the correction data. The control data may comprise instructions by which the laser 12 may be controlled to create the volume body 14.

## Claims

1. A method for providing control data for an ophthalmic device for correction of high order aberrations of an eye, comprising the following steps performed by a control device (18):
- determining a wavefront from a predetermined wavefront measurement;
- determining high degree wavefront aberration coefficients in dependence on the determined wavefront by using a decomposition of the wavefront into a Gatinel-Malet basis,
∘ wherein the Gatinel-Malet basis is formed by a low degree Gatinel-Malet basis that comprises low degree aberration polynomials and a high degree Gatinel-Malet basis that comprises the high degree aberration polynomials,
∘ wherein the aberration polynomials of the low degree Gatinel-Malet basis are orthogonal to each other and the aberration polynomials of the high degree Gatinel-Malet basis are orthogonal to each other, and
∘ wherein the aberration polynomials of the low degree Gatinel-Malet basis are non-orthogonal to the aberration polynomials of the high degree Gatinel-Malet basis;
- determining correction data for the eye by using the determined high degree wavefront aberration coefficients;
- providing control data for the ophthalmic device, comprising the determined correction data.

2. The method according to claim 1, comprising the steps of:
- determining manifest refraction values from predetermined subjective examination data;
- determining low degree manifest aberration coefficients by fitting the manifest refraction values to the low degree aberration polynomials of the low degree Gatinel-Malet basis, wherein the low degree manifest aberration coefficients of the low degree aberration polynomials represent a low degree manifest aberration wavefront;
- subtracting the obtained low degree manifest aberration wavefront from the determined wavefront to obtain a high degree aberration wavefront;
- decomposing the high degree aberration wavefront by using the high degree aberration polynomials of the high degree Gatinel-Malet basis to obtain high degree wavefront aberration coefficients;
- determining the correction data for the eye by using the obtained high degree wavefront aberration coefficients.

3. The method according to claim 1, comprising the steps of:
- determining manifest refraction values from predetermined subjective examination data;
- determining low degree manifest aberration coefficients by fitting the manifest refraction values to the low degree aberration polynomials of the low degree Gatinel-Malet basis;
- determining low degree wavefront aberration coefficients from the wavefront by using the low degree aberration polynomials of the low degree Gatinel-Malet basis;
- calculating a difference between the low degree manifest aberration coefficients and the low degree wavefront aberration coefficients to obtain low degree delta coefficients;
- converting the low degree delta coefficients into the high degree Gatinel-Malet basis to obtain high degree delta coefficients;
- adding the obtained high degree delta coefficients to the high degree wavefront aberration coefficients to obtain manifest corrected high degree wavefront aberration coefficients;
- determining the correction data by using the obtained manifest corrected high degree wavefront aberration coefficients.

4. The method according to claim 1, comprising the steps of:
- determining manifest refraction values from predetermined subjective examination data;
- determining low degree manifest aberration coefficients by fitting the manifest refraction values to the low degree aberration polynomials of the low degree Gatinel-Malet basis;
- determining low degree wavefront aberration coefficients from the wavefront by using the low degree aberration polynomials of the low degree Gatinel-Malet basis;
- calculating a difference between the low degree manifest aberration coefficients and the low degree wavefront aberration coefficients to obtain low degree delta coefficients;
- fitting the low degree delta coefficients by using the aberration polynomials of the low and high degree Gatinel-Malet basis with the constraint that the low degree fit coefficients of the low degree aberration polynomials result in Zero or a predefined range around Zero, to obtain high degree delta coefficients;
- adding the obtained high degree delta coefficients to the high degree wavefront aberration coefficients to obtain manifest corrected high degree wavefront aberration coefficients;
- determining the correction data by using the obtained manifest corrected high degree wavefront coefficients.

5. The method according to claim 1, comprising the steps of:
- fitting the determined wavefront by using the low and high degree aberration polynomials of the Gatinel-Malet basis to obtain low and high degree aberration coefficients, wherein the high degree aberration coefficients of the high degree aberration polynomials represent a high degree aberration wavefront;
- fitting the high degree aberration wavefront by using the low degree polynomials of the low degree Gatinel-Malet basis to obtain coefficients of a low degree portion of the high degree aberration wavefront;
- subtracting the coefficients of the low degree portion from the high degree wavefront aberration coefficients to obtain corrected high degree wavefront aberration coefficients;
- determining the correction data using the obtained corrected high degree wavefront aberration coefficients.

6. The method according to claim 1, comprising the steps of:
- determining manifest refraction values from predetermined subjective examination data;
- determining low degree manifest aberration coefficients by fitting the manifest refraction values to the low degree aberration polynomials of the low degree Gatinel-Malet basis;
- decomposing the wavefront by using only the low degree aberration polynomials of the low degree Gatinel-Malet basis to obtain low degree wavefront aberration coefficients;
- calculating a difference between the low degree manifest aberration coefficients and the low degree wavefront aberration coefficients to obtain low degree delta coefficients;
- fitting the wavefront to low and high degree aberration polynomials using the low and high degree Gatinel-Malet basis, wherein the low degree delta coefficients are accounted for in the fit as a dynamic fit constraint;
- determining high order aberration correction data using the obtained high degree wavefront aberration coefficients.

7. The method according to claim 1, comprising the steps of:
- determining manifest refraction values from predetermined subjective examination data;
- determining low degree manifest aberration coefficients by fitting the manifest refraction values to the low degree aberration polynomials of the low degree Gatinel-Malet basis;
- fitting the wavefront to low and high degree aberration polynomials using the low and high degree Gatinel-Malet basis with the constraint that the low degree fit coefficients of the low degree aberration polynomials result in the determined low degree manifest aberration coefficients, to obtain manifest corrected high degree wavefront coefficients
- determining the correction data by using the obtained manifest corrected high degree wavefront coefficients.

8. A control device (18), configured to perform a method according to any of the preceding claims.

9. A treatment apparatus (10) with at least one ophthalmological laser (12) for the correction of high order aberrations of a human or animal eye by optical breakdown and with at least one control device (18) according to claim 8.

10. A computer program including instructions that cause a treatment apparatus (10) according to claim 9 to execute the method steps according to any of the claims 1 to 7.

11. A non-transitory computer-readable medium, on which the computer program according to claim 10 is stored.
